# EUROPÄISCHE PATENTSCHRIFT

(11) **EP 0 690 031 B1**
(45) Veröffentlichungstag und Bekanntmachung des Hinweises auf die Patenterteilung: **19.03.1997**
(21) Anmeldenummer: 95250156.7
(22) Anmeldetag: 29.06.1995
(51) Int. Cl.: C03C 10/00, A61K 6/06

(54) **Zr02-haltige Glaskeramik**
Zro2-containing glass ceramics
Vitrocéramiques contenant Zro2

(30) Priorität: 01.07.1994 DE 4423794
(43) Veröffentlichungstag der Anmeldung: 03.01.1996
(73) Patentinhaber: IVOCLAR AG, FL-9494 Schaan (LI)
(72) Erfinder: Frank, Martin, FL-9494 Schaan (LI); Rheinberger, Volker, FL-9490 Vaduz (LI); Höland, Wolfram, FL-9494 Schaan (LI)
(74) Vertreter: UEXKÜLL & STOLBERG

(56) Entgegenhaltungen:
- EP-A- 0 518 454
- DE-A- 4 020 893
- CHEMICAL ABSTRACTS, vol. 107, no. 18, November 1987 Columbus, Ohio, US; abstract no. 160108, Seite 373;

## Beschreibung

Die Erfindung betrifft eine ZrO₂-haltige Glaskeramik, die sich aufgrund ihrer ausgezeichneten mechanischen Eigenschaften und ihrer Verarbeitbarkeit bei niedrigen Temperaturen insbesondere zum Einsatz in der Dentaltechnik eignet.

ZrO₂-haltige Glaskeramiken sind bekannt. In geringen Konzentrationen von bis zu 5 Gew.-% wird ZrO₂ in herkömmlichen Glaskeramiken als Keimbildner eingesetzt. Glaskeramiken mit Gehalten von bis zu 15 Gew.-% ZrO₂ sind im Stand der Technik ebenfalls beschrieben. Sie enthalten jedoch kein Phosphorpentoxid und kein oder nur sehr geringe Mengen an Lithiumoxid.

So werden beispielsweise in der US-P 4,687,749 Glaskeramiken mit Enstatit als Hauptkristallphase beschrieben, die einen ZrO₂-Gehalt von bis zu 15 Gew.-% haben können. Die Glaskeramiken sind frei von Phosphoroxid und können nur geringe Mengen an Lithiumoxid, und zwar bis zu 2 Gew.-% enthalten. Weiter handelt es sich um sehr schwer schmelzbare Materialien, so daß zu ihrer Verarbeitung hohe Temperaturen von mehr als 1200°C und insbesondere von etwa 1500°C erforderlich sind.

Aus der DE-OS 42 07 180 sind ZrO₂-haltige Glaskeramiken bekannt, die sich zur Herstellung von Zahnkronen eignen, aber keinerlei Phosphoroxid oder Lithiumoxid aufweisen. Der ZrO₂-Gehalt ist auf maximal 15 Gew.-% beschränkt, da es sonst schwierig ist, ein homogenes Glas zu erhalten.

Neben ZrO₂-haltigen Glaskeramiken, die im Ausgangsglas ZrO₂ glasig homogen gelöst enthalten und bei denen ZrO₂ Kristalle durch thermische Behandlung des Ausgangsglases nachträglich kristallisieren, sind auch Sinterprodukte bzw. Sinterkörper mit ZrO₂ bekannt. Bei diesen Sinterprodukten wird kristallines ZrO₂ in Pulverform zu einem Glaspulver gemischt und durch Sinterreaktion zu Produkten verarbeitet.

So sind beispielsweise aus der DE-PS 39 05 895 Cordierit-Sinterkörper mit bis zu 50 Gew.-% ZrO₂ bekannt, die aber keinerlei Phosphoroxid oder Lithiumoxid enthalten. Das ZrO₂ wird nicht durch gesteuerte Kristallisation eines entsprechenden Ausgangsglases erhalten, sondern in Pulverform zugegeben. Durch Sinterung des ZrO₂-Pulvers mit gepulvertem Ausgangsglas werden die gewünschten Cordierit-ZrO₂-Sinterprodukteerzeugt. Nachteilig an den beschriebenen Produkten ist, daß sie nicht durch das für die Dentaltechnik vorteilhafte Verfahren des viskosen Fließens unterhalb von 200° C weiterverarbeitet werden können.

Der Erfindung liegt somit die Aufgabe zugrunde, Glaskeramiken mit einem hohen ZrO₂-Gehalt zu schaffen, die über ein sehr gute mechanische Festigkeit verfügen, bei niedrigen Temperaturen von weniger als 1200°C verarbeitbar, insbesondere durch Pressen verformbar sind, und einen sehr guten Haftverbund zu gesinterten ZrO₂-Keramiken ausbilden und demgemäß in vorteilhafter Weise als Werkstoffe für geformte Dentalprodukte eingesetzt werden können.

Diese Aufgabe wird durch die ZrO₂-haltige Glaskeramik nach den Ansprüchen 1 bis 5 gelöst. Die Erfindung betrifft ebenfalls ein Verfahren zur Herstellung der Glaskeramik nach den Ansprüchen 6 bis 9, ihre Verwendung nach den Ansprüchen 10 bis 12 sowie geformte Dentalprodukte nach Anspruch 13, die einen Gehalt an der Glaskeramik aufweisen.

Die erfindungsgemäße ZrO₂-haltige Glaskeramik ist dadurch gekennzeichnet, daß sie die folgenden Komponenten enthält:

| Komponente | Gew.-% |
|---|---|
| SiO₂ | 42,5 bis 58,5 |
| Al₂O₃ | 0 bis 7,0 |
| La₂O₃ | 0 bis 9,5 |
| Li₂O | 7,0 bis 14,5 |
| Na₂O | 0 bis 7,5 |
| K₂O | 0 bis 13,5 |
| P₂O₅ | 4,0 bis 13,5 |
| ZrO₂ | 15,0 bis 28,0 |
| TiO₂ | 0 bis 6,0 |
| F | 0 bis 2,0 |
| BaO | 0 bis 6,5 |
| CaO | 0 bis 6,0 |
| B₂O₃ | 0 bis 3,3 |
| CeO₂ | 0 bis 3,0 |

und daß eine ZrO₂-Kristallphase und mindestens eine weitere Kristallphase vorliegen. Vorzugsweise besteht die Glaskeramik im wesentlichen aus den genannten Komponenten.

Für einige der Komponenten existieren besonders bevorzugte Mengenbereiche, und diese sind unabhängig voneinander wählbar und wie folgt:

| | |
|---|---|
| SiO₂ | 47 - 55 Gew.-% |
| Al₂O₃ | 0 - 5 Gew.-% |
| Li₂O | 7 - 14 Gew.-% |
| Na₂O | 0 - 6 Gew.-% |
| K₂O | 0 - 8 Gew.-% |
| P₂O₅ | 5 - 11 Gew.-% |
| ZrO₂ | 16 - 25 Gew.-% |
| F | 0 - 1,5 Gew.-% |

Zur Herstellung der erfindungsgemäßen Glaskeramik wird so vorgegangen, daß man zunächst in herkömmlicher Weise ein Ausgangsglas herstellt, welches die erforderlichen Komponenten enthält. Üblicherweise werden dazu geeignete Ausgangsmaterialien, z.B. Oxide, Oxyhydroxide, Carbonate und/oder Phosphate, bei Temperaturen von 1550 bis 1600° C bis zur Erzielung einer homogenen Glasschmelze erschmolzen.

Das hergestellte Ausgangsglas wird anschließend einer Wärmebehandlung unterzogen, durch die eine gesteuerte Kristallisation herbeigeführt und demgemäß die Glaskeramik gebildet wird. Zur Durchführung der Wärmebehandlung und eines vorherigen etwaigen Formgebungsschrittes gibt es mehrere Möglichkeiten.

Beispielsweise kann das erschmolzene Ausgangsglas zuerst zu einem gewünschten Formkörper gegossen werden, der anschließend der eigentlichen Wärmebehandlung durch Erwärmen auf eine Temperatur im Bereich von 580 - 1100° C für 0,5 bis 2 Stunden unterzogen wird.

Weiter ist es möglich, daß man (a) entweder durch Gießen von geschmolzenem Ausgangsglas oder (b) durch uniaxiales oder isostatisches Kaltpressen und anschließendes Sintern von granuliertem Ausgangsglas einen gewünschten Formkörper bildet und diesen im viskosen Zustand bei einer Temperatur von 850 - 1200° C verpreßt. Dabei erfolgt die Kristallisation des Ausgangsglases
(1) durch das Sintern des Ausgangsglases,
(2) durch das Heißpressen bei 850 - 1200° C und
(3) durch eine nach dem Heißpressen oder gegebenenfalls noch durchgeführte weitere Wärmebehandlung.

Die hier angegebene Möglichkeit der Wärmebehandlung durch Heißpressen verdeutlicht gleichzeitig die besondere Vorteilhaftigkeit der erfindungsgemäßen Glaskeramik gegenüber herkömmlichen hochfesten Glaskeramikmaterialien, die bei so niedrigen Temperaturen nicht zu Formkörpern verpreßt werden können. Das Verpressen im viskosen Zustand wird vorzugsweise unter Verwendung des in EP-A-0 231 773 beschriebenen Verfahrens und Preßofens durchgeführt.

In einer bevorzugten Ausführungsform kann man, wie oben bereits erwähnt, nach der Wärmebehandlung durch Pressen im viskosen Zustand eine weitere Wärmebehandlung im Temperaturbereich von 580 - 1100° C durchführen
Es hat sich herausgestellt, daß bei Erhöhung des Anteils von ZrO₂ in dem Ausgangsglas auf mehr als 28 Gew.-% eine homogene Glasschmelze nicht mehr erzielt werden kann. Offensichtlich liegt in diesem Bereich die Löslichkeitsgrenze für das erfindungsgemäß eingesetzte Glassystem. So lag bei der Verwendung von 33 Gew.-% ZrO₂ nach dem Erschmelzen noch ungelöstes ZrO₂ vor. In einem solchen Fall können die erfindungsgemäßen Glaskeramiken nicht gebildet werden, die gerade durch gesteuerte Entglasung eines homogenen Ausgangsglases unter Bildung von hochdispers verteilten ZrO₂-Kristallen erhalten werden.

Mittels rasterelektronenemikroskopischer Untersuchungen wurden die erfindungsgemäßen Glaskeramiken näher analysiert. Dabei zeigte sich, daß sich diese durch ein charakteristisches Gefüge aus unterschiedlich großen Kristallen auszeichnen. Typischerweise haben die Kristalle eine mittlere Größe von 20 µm bezogen auf die Anzahl der Kristalle. Bevorzugt sind jedoch Kristallgrößen von kleiner oder gleich 5 µm, insbesondere kleiner oder gleich 1 µm. Es ist möglich, daß zwischen diesen Kristallen noch feinkristalline Partikel mit einer Größe von weniger als etwa 400 nm vorliegen, welche sich nahezu berühren oder in direktem Kontakt miteinander befinden.

Aus den Ergebnissen von Röntgenbeugungstests und Materialkontrastuntersuchungen im Rasterelektronenmikroskop wird geschlossen, daß die größeren Kristalle durch eine SiO₂-Modifikation, insbesondere Cristobalit, und/oder Lithiumphosphat gebildet sind. Bei den kleineren Kristallen handelt es sich um ZrO₂-Kristalle, insbesondere als Baddeleyit und/oder in tetragonaler Form, welche vorzugsweise die Hauptkristallphase bilden, und um Lithiumsilikat-Kristalle (Li₂SiO₃). In einzelnen Fällen sind in den erfindungsgemäßen Glaskeramiken auch Aluminiumphosphat (AlPO₄), Lithiumaluminiumsilikat und in geringer Konzentration sogar Lithiumzirkonsilikat (Li₂ZrSi₆O₁₅) vorhanden.

Bevorzugt enthält die ZrO₂-Kristallphase ZrO₂ in tetragonaler Form. Die tetragonale Modifikation der ZrO₂-Kristalle ist deshalb bevorzugt, weil durch sie bei Einwirkung einer äußeren Kraft, z.B. einer Rißausbreitung eine Umwandlung zur monoklinen Modifikation erfolgen kann. Die Modifikationsumwandlung führt zum bekannten Effekt der Hemmung der Rißfortpflanzung und damit zur Festigkeits- bzw. Bruchzähigkeitssteigerung.

Das vorstehend skizzierte Gefüge ist typisch für die erfindungsgemäßen Glaskeramiken, unabhängig davon, ob sie als gegossene oder verpreßte Glaskeramiken vorliegen. Unterschiede treten lediglich hinsichtlich des Volumenanteils der einzelnen Kristallphasen sowie der Größe der Kristallite der einzelnen Phasen auf. Es wird angenommen, daß die einzelnen Kristallphasen durch einen dispersionsverstärkenden Effekt zur Steigerung der Festigkeit der erfindungsgemäßen Glaskeramiken führen. In welcher Weise dies genau erfolgt, ist derzeit noch unklar. In diesem Zusammenhang ist es außerdem als überraschend zu bezeichnen, daß der Einbau von Cristobalit trotz der bekannten Unterschiede in den Ausdehnungskoeffizienten seiner Modifikationen keine negative Beeinflussung der Festigkeit bewirkt.

Aufgrund ihrer speziellen chemischen Zusammensetzung und ihres besonderen Gefüges weisen die erfindungsgemäßen Glaskeramiken mehrere Vorteile auf, die sie besonders als Dentalwerkstoff geeignet machen. Zum einen besitzen sie sehr hohe Biegebruchfestigkeiten von bis zu 400 MPa. Darüber hinaus zeigen sie eine gute Temperaturwechselbeständigkeit und können entweder mit hohem Weißheitsgrad, infolge des hohen Gehalts an ZrO₂-Kristallen, oder in transluzenter Form erhalten werden, was für Dentalmaterialien und die aus ihnen hergestellten geformten Dentalprodukte von besonderer Bedeutung ist. Weiter ist aber auch eine Einfärbung der erfindungsgemäßen Glaskeramiken möglich. Dies kann a) durch die Einfärbung des Ausgangsglases durch Zusatz von Oxiden der 3 d-Elemente und/oder der 4 f-Elemente und/oder durch Metallkolloide oder b) durch Zugabe von Farbpigmenten zum Ausgangsglasgranulat erfolgen. Ebenso ist es möglich, Fluoreszenzmittel zuzusetzen. Die Glaskeramiken können auch noch weitere übliche Zusatzstoffe enthalten, sofern diese die Kristallisation des Ausgangsglases nicht behindern.

Außerdem sind die erfindungsgemäßen Glaskeramiken auch bei Temperaturen von unterhalb 1200°C verarbeitbar, wozu insbesondere das für die Herstellung von Dentalprodukten vorteilhafte Heißpreßverfahren im viskosen Zustand eingesetzt wird. Eine Formung von herkömmlichen hochfesten Glaskeramikmaterialien ist bei diesen niedrigen Temperaturen häufig nicht möglich. Weiterhin ist es ein besonderer Vorteil, daß die erfindungsgemäßen Glaskeramiken im Gegensatz zu konventionellen Glaskeramiken auch nicht mit der Einbettmasse reagieren, die bei der Herstellung von geformten Dentalprodukten mittels Heißpreßverfahren eingesetzt wird. Dies ist ein wesentlicher Vorteil für den sie verarbeitenden Dentaltechniker.

Schließlich haften die erfindungsgemäßen Glaskeramiken sehr gut an hochfesten reinen ZrO₂-Keramiken, was besonders für die Verwendung in der Dentaltechnik wichtig ist. So kann z.B. an einem hochfesten ZrO₂-Keramik-Wurzelstift direkt nach individueller Formgebung, das heißt, in Abhängigkeit von der jeweiligen Kavität, eine paßfähige ZrO₂-Glaskeramik angepreßt werden. Damit wird der hochfeste ZrO₂-Keramik-Zahnwurzelstift fest im Zahn verankert und ein weiterer Zahnaufbau kann vorgenommen werden.

Angesichts der vorstehend geschilderten Eigenschaften wird die erfindungsgemäße Glaskeramik auch vorzugsweise als (a) Dentalmaterial oder daraus geformtes Dentalprodukt oder als (b) Bestandteil von Dentalmaterial oder von daraus geformtem Dentalprodukt eingesetzt. Bevorzugte Dentalprodukte sind dabei Zahnwurzelkonstruktionen, insbesondere Zahnwurzelstifte oder Zahnwurzelaufbauten.

Im folgenden wird die Erfindung anhand von Beispielen näher erläutert.

### Beispiele

### Beispiele 1 bis 18

Es wurden insgesamt 18 verschiedene erfindungsgemäße Glaskeramiken hergestellt. Sie hatten die in der Tabelle I angegebenen chemischen Zusammensetzungen.

Für einige dieser Glaskeramiken finden sich in Tabelle II neben Hinweisen zum dem jeweils ausgewählten Herstellungsverfahren auch Angaben zu verschiedenen Eigenschaften. Die angegebenen Werte für die Biegefestigkeit sind Mittelwerte aus mehreren Messungen gemäß ISO 6872-1984, und die gemessenen Werte wichen um maximal ± 30 bis 40 MPa von diesen Mittelwerten ab. Es wird darauf hingewiesen, daß in vielen Fällen neben den in der Tabelle angegebenen Kristallphasen noch weitere Kristallphasen in zum Teil geringer Konzentration und Größe auftraten, die jedoch röntgenographisch nicht eindeutig identifiziert werden konnten.

Beispiele für gegossene Glaskeramiken stellen Nr. 1 und 6 dar, und diese wurden entsprechend dem in Beispiel 19 beschriebenen Verfahren hergestellt, wobei die jeweils verwendete Wärmebehandlung aus der Tabelle II hervorgeht.

Beispiele für verpreßte Glaskeramiken sind Nr. 3, 7, 10 und 11, und diese wurden entsprechend dem in Beispiel 20 beschriebenen Verfahren erhalten. Dabei wurden die Glaskeramiken Nr. 3, 7, 10 und 11 gemäß Variante A (Gießen, Feinabkühlen, Verpressen im viskosen Zustand) und die Glaskeramik Nr. 3 gemäß Variante B (Fritten, Kaltpressen, Sintern, Verpressen im viskosen Zustand) hergestellt. Sofern nach dem Verpressen im viskosen Zustand noch eine weitere Wärmebehandlung durchgeführt wurde, so ist dies in der Tabelle als "Thermische Nachbehandlung" angegeben.

Die Beispiele verdeutlichen wie durch Veränderung der chemischen Zusammensetzung des Ausgangsglases und des Herstellungsverfahrens Glaskeramiken mit unterschiedlichem Gefüge und Eigenschaften erhalten werden können.

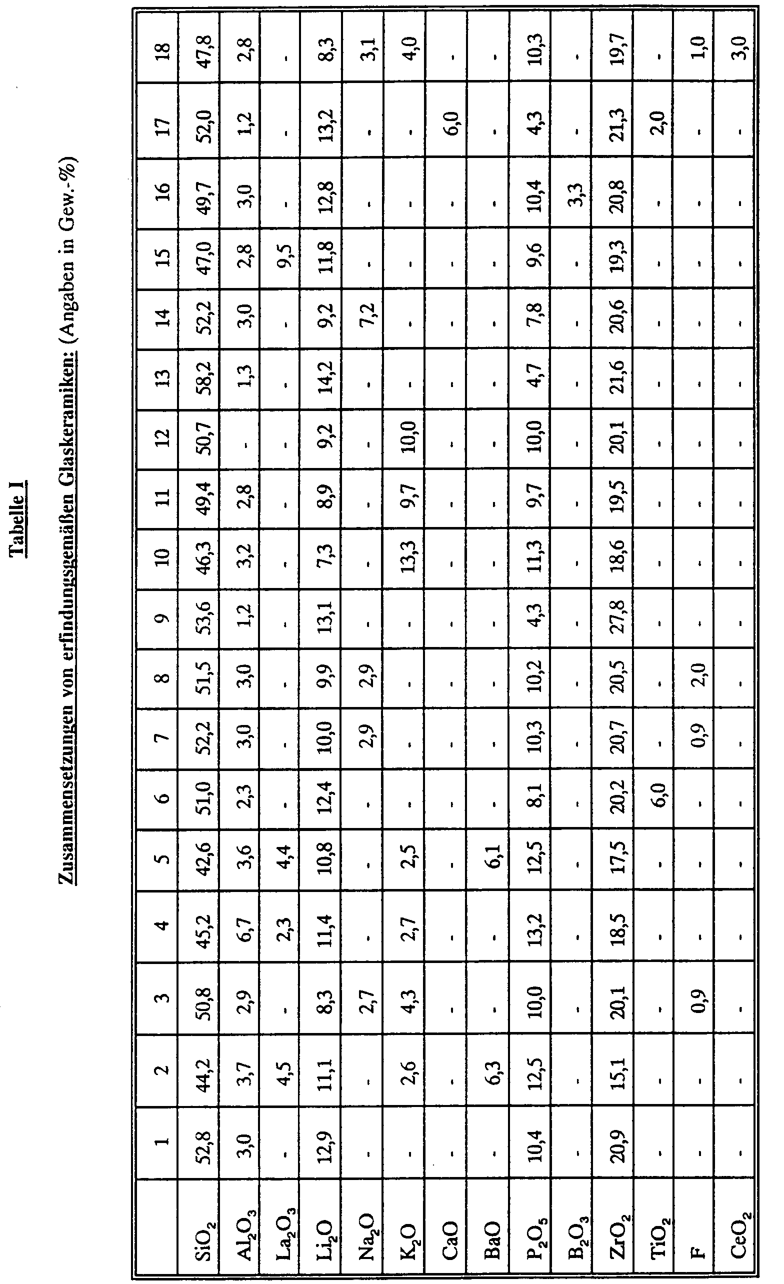

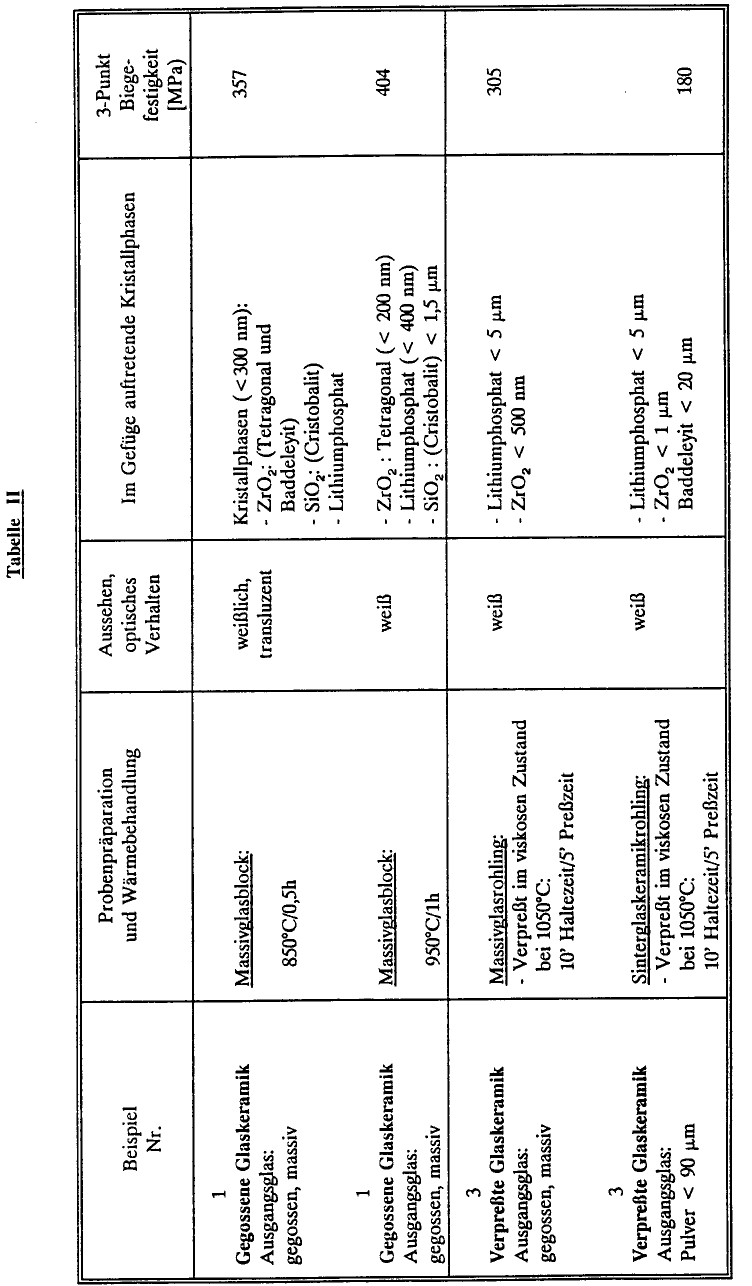

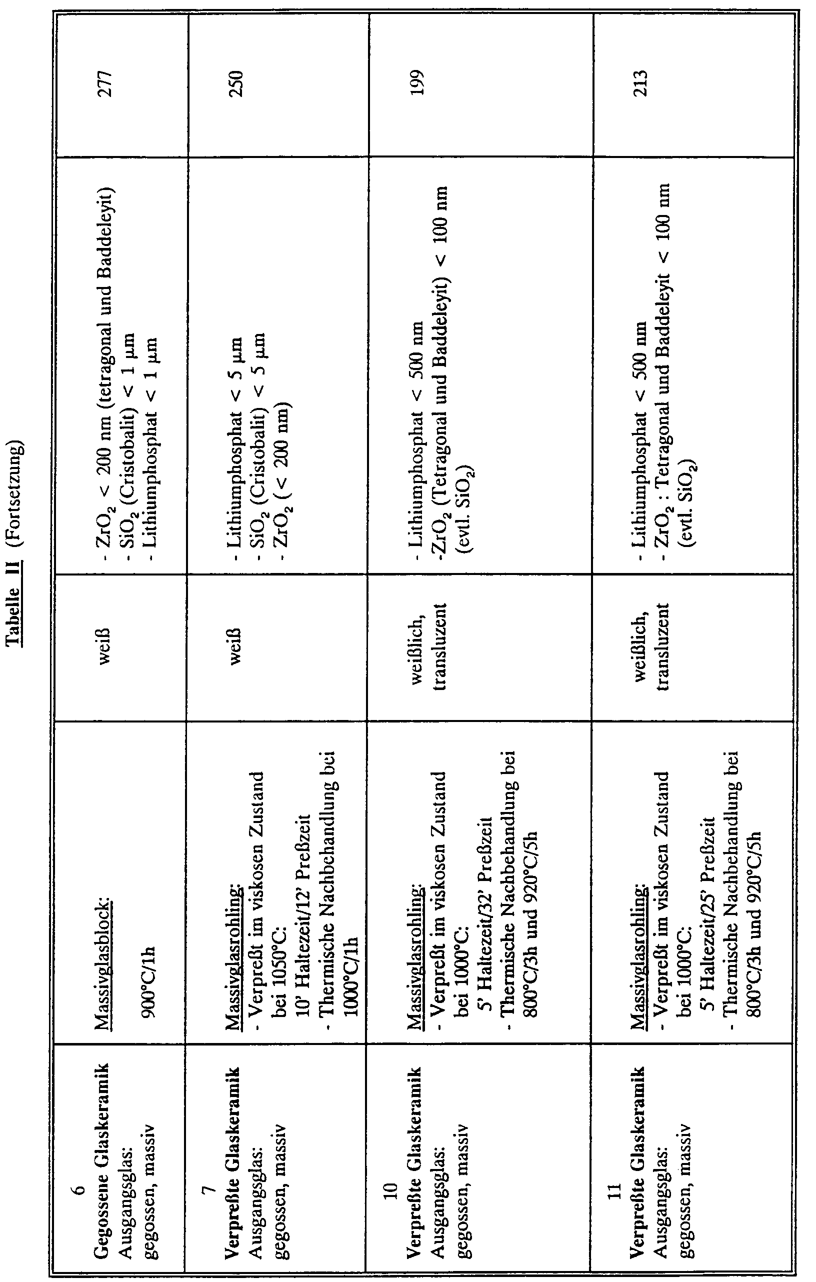

### Beispiel 19 - Gegossene Glaskeramik

Es wurde zunächst ein Ausgangsglas mit der in Tabelle I für die Glaskeramik Nr. 1 angegebenen chemischen Zusammensetzung hergestellt. Dazu wurde ein entsprechendes Gemisch von Oxiden, Oxyhydroxiden, Carbonaten und Phosphaten in einem Platin/Rhodium-Tiegel 2 Stunden lang bei Temperaturen von 1500 bis 1600° C erschmolzen. Das erhaltene geschmolzene Glas wurde in Wasser abgeschreckt, d.h. gefrittet, getrocknet, granuliert und erneut 2 Stunden lang bei 1500 bis 1600° C erschmolzen, um eine gute Homogenität zu erzielen.

Die Glasschmelze wurde dann zu einem Massivglasblock mit einem Gewicht von ca. 100g gegossen, und der Glasblock wurde ab 650°C so langsam abgekühlt, daß keine Spannungen im Glas entstanden. Aus dem Glasblock wurden Stäbe (ca. 5 x 2 x 25mm) geschnitten, welche 30 Minuten lang bei 850°C wärmebehandelt wurden. Die für die erhaltenen Glaskeramikstäbe gemessene Biegefestigkeit sowie weitere Eigenschaften der Glaskeramik und Angaben zu deren Gefüge sind in Tabelle II aufgeführt.

Die in diesem Beispiel hergestellte Glaskeramik eignet sich u.a. aufgrund ihrer hohen Festigkeit, Transluzenz und einfachen Verarbeitbarkeit sowie ihres weißen Aussehens in ausgezeichneter Weise als Dentalmaterial, welches z.B beim Erstellen eines Zahnwurzelaufbaus eingesetzt werden kann.

### Beispiel 20 - Verpreßte Glaskeramiken

Es wurde zunächst ein Ausgangsglas mit der in Tabelle I für Glaskeramik Nr. 3 angegebenen chemischen Zusammensetzung hergestellt. Dazu wurde eine Schmelze des Ausgangsglases wie in Beispiel 19 durch zweimaliges Erschmelzen erzeugt. Die erhaltene Schmelze wurde dann nach zwei unterschiedlichen Varianten (A) und (B) weiterverarbeitet.

Variante (A): Hierbei wurde die Schmelze des Ausgangsglases zu einem Massivglasstab (Durchmesser: 11,3 mm; Länge: 50 mm) gegossen und langsam abgekühlt, um das Entstehen von Spannungen zu vermeiden. Dann wurde aus dem Stab ein kleiner zylindrischer Massivglasrohling (Durchmesser: 11,3 mm; Länge: 15 mm) herausgeschnitten, und dieser wurde unter Verwendung des Preßverfahrens und Preßofens gemäß EP-A-0 231 773 unter Vakuum und bei einer Temperatur von 1050° C im viskosen Zustand zu der gewünschten Probengeometrie bei 5 bar Preßdruck verpreßt.

Es ist ein besonderer Vorteil dieser Glaskeramik gegenüber herkömmlichen Materialien, daß sie selbst bei Temperaturen von weniger als 1200° C, nämlich bei 1050° C, zu individuell geformten Dentalprodukten, verarbeitbar ist, z.B. an hochfeste ZrO₂-Keramik-Zahnwurzelstifte, angepreßt werden kann. Dabei führt das Verpressen auch nicht zu einer bei konventionellen Materialien häufig auftretenden unerwünschten Reaktion mit der erforderlichen Einbettmasse, was die ausgezeichnete Eignung der erfindungsgemäßen Glaskeramik zur Herstellung von individuell geformten Dentalprodukten unterstreicht.

Variante (B): Hierbei wurde zunächst die Schmelze des Ausgangsglases durch Eingießen in Wasser gefrittet, und die entstandene Fritte wurde gemahlen und auf eine Korngröße von weniger als 90 µm gesiebt. Anschließend wurde das erhaltene Glaspulver mittels einer uniaxialen Trockenpresse bei 1000 bar Preßdruck zu kleinen Zylindern verpreßt. Die Glaszylinder wurden dann im Vakuum bei einer Temperatur von 850° C 30 Minuten lang in einem Brennofen gesintert, wodurch bereits in einem gewissen Umfang die Entglasung des Ausgangsglases erfolgte. Die erhaltenen Rohlinge wurden schließlich unter Verwendung des Preßverfahrens und Preßofens gemäß EP-A-0 231 773 unter Vakuum im viskosen Zustand zu der gewünschten Probengeometrie verpreßt.

Auch die auf diese Weise erhaltene erfindungsgemäße Glaskeramik wies die bei Variante (A) erhaltenen Vorzüge bei der Verarbeitung auf und konnte somit in vorteilhafter Weise z.B. zur Herstellung von individuell geformten Aufbauten für Zahnwurzelstifte verwendet werden.

## Patentansprüche

1. ZrO₂-haltige Glaskeramik, **dadurch gekennzeichnet**, daß sie die folgenden Komponenten enthält:
| Komponente | Gew.-% |
|---|---|
| SiO₂ | 42,5 bis 58,5 |
| Al₂O₃ | 0 bis 7,0 |
| La₂O₃ | 0 bis 9,5 |
| Li₂O | 7,0 bis 14,5 |
| Na₂O | 0 bis 7,5 |
| K₂O | 0 bis 13,5 |
| P₂O₅ | 4,0 bis 13,5 |
| ZrO₂ | 15,0 bis 28,0 |
| TiO₂ | 0 bis 6,0 |
| F | 0 bis 2,0 |
| BaO | 0 bis 6,5 |
| CaO | 0 bis 6,0 |
| B₂O₃ | 0 bis 3,3 |
| CeO₂ | 0 bis 3,0 |
und daß eine ZrO₂-Kristallphase und mindestens eine weitere Kristallphase vorliegen.

2. Glaskeramik nach Anspruch 1, **dadurch gekennzeichnet**, daß die Mengen von einigen Komponenten unabhängig voneinander wie folgt sind:
| | |
|---|---|
| SiO₂ | 47 - 55 Gew.-% |
| Al₂O₃ | 0 - 5 Gew.-% |
| Li₂O | 7 - 14 Gew.-% |
| Na₂O | 0 - 6 Gew.-% |
| K₂O | 0 - 8 Gew.-% |
| P₂O₅ | 5 - 11 Gew.-% |
| ZrO₂ | 16 - 25 Gew.-% |
| F | 0 - 1,5 Gew.-% |

3. Glaskeramik nach Anspruch 1 oder 2, **dadurch gekennzeichnet**, daß die ZrO₂-Kristallphase ZrO₂ in tetragonaler Modifikation enthält.

4. Glaskeramik nach einem der Ansprüche 1 bis 3, **dadurch gekennzeichnet**, daß eine weitere Kristallphase durch eine SiO₂-Kristallmodifikation und/oder eine zu SiO₂ isotype AlPO₄-Modifikation gebildet ist.

5. Glaskeramik nach einem der Ansprüche 1 bis 4, **dadurch gekennzeichnet**, daß eine weitere Kristallphase durch Lithiumphosphat gebildet ist.

6. Verfahren zur Herstellung einer Glaskeramik gemäß einem der Ansprüche 1 bis 5, **dadurch gekennzeichnet**, daß man
(a) ein Ausgangsglas herstellt, welches die Komponenten gemäß Anspruch 1 enthält, und
(b) das Ausgangsglas einer Wärmebehandlung unterzieht, wodurch die Glaskeramik gebildet wird.

7. Verfahren nach Anspruch 6, **dadurch gekennzeichnet**, daß man durch Gießen von geschmolzenem Ausgangsglas oder durch Sintern von granuliertem Ausgangsglas einen Formkörper bildet und diesen in Schritt (b) durch Pressen im viskosen Zustand bei einer Temperatur von 850° C bis 1200° C wärmebehandelt.

8. Verfahren nach Anspruch 6 oder 7, **dadurch gekennzeichnet**, daß man nach der Wärmebehandlung durch Pressen im viskosen Zustand eine weitere Wärmebehandlung bei 580 - 1100°C durchführt.

9. Verfahren nach Anspruch 6, **dadurch gekennzeichnet**, daß man durch Gießen von geschmolzenem Ausgangsglas einen Formkörper bildet und diesen in Schritt (b) einer Wärmebehandlung bei 850 - 1100° C unterzieht.

10. Verwendung der Glaskeramik gemäß einem der Ansprüche 1 bis 5 als (a) Dentalmaterial oder daraus geformtes Dentalprodukt oder als (b) Bestandteil von Dentalmaterial oder von daraus geformtem Dentalprodukt.

11. Verwendung nach Anspruch 10, wobei das geformte Dentalprodukt eine Zahnwurzelkonstruktion ist.

12. Verwendung nach Anspruch 11, wobei die Zahnwurzelkonstruktion ein Zahnwurzelstift oder ein Zahnwurzelaufbau ist.

13. Geformtes Dentalprodukt, welches einen Gehalt an der Glaskeramik gemäß einem der Ansprüche 1 bis 5 aufweist.

## Claims

1. Glass ceramic containing ZrO₂, characterized in that it contains the following components:
| Component | % by weight |
|---|---|
| SiO₂ | 42.5 to 58.5 |
| Al₂O₃ | 0 to 7.0 |
| La₂O₃ | 0 to 9.5 |
| Li₂O | 7.0 to 14.5 |
| Na₂O | 0 to 7.5 |
| K₂O | 0 to 13.5 |
| P₂O₅ | 4.0 to 13.5 |
| ZrO₂ | 15.0 to 28.0 |
| TiO₂ | 0 to 6.0 |
| F | 0 to 2.0 |
| BaO | 0 to 6.5 |
| CaO | 0 to 6.0 |
| B₂O₃ | 0 to 3.3 |
| CeO₂ | 0 to 3.0 |
and in that there is a ZrO₂ crystalline phase and at least one other crystalline phase.

2. Glass ceramic according to Claim 1, characterized in that the amounts of certain components, independently of one another, are as follows:
| | |
|---|---|
| SiO₂ | 47 - 55% by weight |
| Al₂O₃ | 0 - 5% by weight |
| Li₂O | 7 - 14% by weight |
| Na₂O | 0 - 6% by weight |
| K₂O | 0 - 8% by weight |
| P₂O₅ | 5 - 11% by weight |
| ZrO₂ | 16 - 25% by weight |
| F | 0 - 1.5% by weight |

3. Glass ceramic according to Claim 1 or 2, characterized in that the ZrO₂ crystalline phase contains ZrO₂ in tetragonal form.

4. Glass ceramic according to one of Claims 1 to 3, characterized in that a further crystalline phase is formed by an SiO₂ crystal form and/or an AlPO₄ form isotypic with SiO₂.

5. Glass ceramic according to one of Claims 1 to 4, characterized in that a further crystalline phase is formed by lithium phosphate.

6. Process for the production of a glass ceramic according to one of Claims 1 to 5, characterized in that:
(a) an initial glass which contains the components according to Claim 1 is produced, and
(b) the initial glass is subjected to a heat treatment, by means of which the glass ceramic is formed.

7. Process according to Claim 6, characterized in that a shaped article is formed by casting initial glass in the molten state or by sintering initial glass in granular form, and this shaped article is heat-treated in step (b) by compression in the viscous state at a temperature of 850°C to 1200°C.

8. Process according to Claim 6 or 7, characterized in that a further heat treatment is carried out at 580 - 1100°C after the heat treatment by compression in the viscous state.

9. Process according to Claim 6, characterized in that a shaped article is formed by casting the initial glass in the molten state, and this shaped article is subjected to a heat treatment in step (b) at 850 - 1100°C.

10. Use of the glass ceramic according to one of Claims 1 to 5 as (a) dental material or a shaped dental product made therefrom, or as (b) a component of dental material or of a shaped dental product made therefrom.

11. Use according to Claim 10, the shaped dental product being a dental root construction.

12. Use according to Claim 11, the dental root construction being a root canal post or a dental root extension.

13. Shaped dental product which contains the glass ceramic according to one of Claims 1 to 5.

## Revendications

1. Vitrocéramique contenant du ZrO₂, caractérisée en ce qu'elle contient les composants suivants :
| COMPOSANTS | % EN POIDS |
|---|---|
| SiO₂ | 42,5 à 58,5 |
| Al₂O₃ | 0 à 7,0 |
| La₂O₃ | 0 à 9,5 |
| Li₂O | 7,0 à 14,5 |
| Na₂O | 0 à 7,5 |
| K₂O | 0 à 13,5 |
| P₂O₅ | 4,0 à 13,5 |
| ZrO₂ | 15,0 à 28,0 |
| TiO₂ | 0 à 6,0 |
| F | 0 à 2,0 |
| BaO | 0 à 6,5 |
| CaO | 0 à 6,0 |
| B₂O₃ | 0 à 3,3 |
| CeO₂ | 0 à 3,0 |
et en ce qu'une phase cristalline de ZrO₂ et au moins une autre phase cristalline sont présentes.

2. Vitrocéramique suivant la revendication 1, caractérisée en ce que les quantités de certains composants indépendamment les uns des autres sont les suivantes :
| | |
|---|---|
| SiO₂ | 47 - 55 % en poids |
| Al₂O₃ | 0 - 5 % en poids |
| Li₂O | 7 - 14 % en poids |
| Na₂O | 0 - 6 % en poids |
| K₂O | 0 - 8 % en poids |
| P₂O₅ | 5 - 11 % en poids |
| ZrO₂ | 16 - 25 % en poids |
| F | 0 - 1,5 % en poids |

3. Vitrocéramique suivant la revendication 1 ou 2, caractérisée en ce que la phase cristalline de ZrO₂ contient du ZrO₂ sous forme de sa variété tétragonale.

4. Vitrocéramique suivant l'une des revendications 1 à 3, caractérisée en ce qu'une autre phase cristalline est formée par une variété cristalline de SiO₂ et/ou par une variété de AlPO₄ isotypique relativement à SiO₂.

5. Vitrocéramique suivant l'une des revendications 1 à 4, caractérisée en ce qu'une autre phase cristalline est formée par du phosphate de lithium.

6. Procédé de production d'une vitrocéramique suivant l'une des revendications 1 à 5, caractérisé en ce que
(a) on produit un verre de base qui contient les composants suivant la revendication 1, et
(b) on soumet le verre de base à un traitement thermique, par lequel la vitrocéramique est formée.

7. Procédé suivant la revendication 6, caractérisé en ce que l'on forme un corps façonné par coulée de verre de base fondu ou par agglomération de verre de base granulé et on soumet ce corps dans l'étape (b) à un traitement thermique par pressage à l'état visqueux à une température de 850° à 1200°C.

8. Procédé suivant la revendication 6 ou 7, caractérisé en ce que l'on conduit un autre traitement thermique à 580-1100°C après le traitement thermique par pressage à l'état visqueux.

9. Procédé suivant la revendication 6, caractérisé en ce que l'on forme un corps façonné par coulée de verre de base fondu et on soumet ce corps dans l'étape (b) à un traitement thermique à 850-1100°C.

10. Utilisation de la vitrocéramique suivant l'une des revendications 1 à 5 comme (a) matériau dentaire ou produit dentaire formé à partir de ce matériau ou (b) comme (b) constituant d'un matériau dentaire ou d'un produit dentaire formé à partir de ce matériau.

11. Utilisation suivant la revendication 10, dans laquelle le produit dentaire façonné est une construction de racine de dent.

12. Utilisation suivant la revendication 11, dans laquelle la construction de racine de dent est un pivot ou une superstructure de racine de dent.

13. Produit dentaire façonné, qui présente une teneur en la vitrocéramique suivant l'une des revendications 1 à 5.
